# EUROPÄISCHE PATENTSCHRIFT

(11) **EP 2 110 153 B1**
(45) Veröffentlichungstag und Bekanntmachung des Hinweises auf die Patenterteilung: **09.02.2011**
(21) Anmeldenummer: 09005140.0
(22) Anmeldetag: 08.04.2009
(51) Int. Cl.: A61M 39/02

(54) **Portkatheter zum Einbringen einer Flüssigkeit in ein Hohlorgan eines menschlichen oder tierischen Körpers**
Port catheter for inserting a fluid into a hollow organ of a human or animal body
Cathéter à port implantable destiné à acheminer un liquide dans un organe creux d'un corps humain ou animal

(30) Priorität: 15.04.2008 AT 21908 U
(43) Veröffentlichungstag der Anmeldung: 21.10.2009
(73) Patentinhaber: A.M.I. Agency for Medical Innovations GmbH, 6800 Feldkirch (AT)
(72) Erfinder: Egle, Walter, 6842 Koblach (AT)
(74) Vertreter: Hofmann, Ralf U.

(56) Entgegenhaltungen:
- EP-A- 0 260 080
- EP-A- 1 736 195
- DE-U1- 8 437 873
- US-A- 5 919 160
- US-A1- 2006 217 668

## Beschreibung

Die Erfindung bezieht sich auf einen Portkatheter zum Einbringen einer Flüssigkeit in ein Hohlorgan eines menschlichen oder tierischen Körpers, mit einer in den menschlichen oder tierischen Körper implantierbaren Porteinheit, die eine innere Kammer aufweist, welcher die in das Hohlorgan des menschlichen oder tierischen Körpers einzubringende Flüssigkeit zuführbar ist, und einem an die Porteinheit angeschlossenen Schlauch, der einen inneren Kanal aufweist, durch den hindurch die Flüssigkeit in das Hohlorgan leitbar ist, wobei zum Manipulieren der Porteinheit bei der Implantation des Portkatheters in den menschlichen oder tierischen Körper ein mit der Porteinheit verbundenes und von dieser abnehmbares Griffstück vorgesehen ist.

Portkatheter dienen zum Einbringen von Flüssigkeiten in Hohlorgane des menschlichen oder tierischen Körpers, beispielsweise zur medikamentösen Behandlung. So werden z.B. in der Chemotherapie Medikamente direkt in den Blutkreislauf eingebracht. Abgesehen von der Einbringung von Flüssigkeiten in Blutgefäße können mit Portkathetern Flüssigkeiten auch in den Magen-Darm-Trakt, die Blase und andere Hohlorgane eingebracht werden.

Portkatheter besitzen üblicherweise eine topfartige Porteinheit mit einer von einer Silikonmembran verschlossenen Kammer und einen an die Porteinheit angeschlossenen Schlauch. Die in das Hohlorgan einzubringende Flüssigkeit wird durch Durchstechen der Silikonmembran in die Kammer der Porteinheit eingespritzt und gelangt durch den inneren Kanal des Schlauchs, der mit seinem Abgabeende im Hohlorgan angeordnet ist, in das Hohlorgan. Üblicherweise wird die Porteinheit subkutan implantiert.

Eine herkömmliche Operationsmethode zur Implantation des Portkatheters ist die Seldinger-Technik. Hierbei wird eine Nadel mit einer darauf angeordneten Hülse in das Hohlorgan, insbesondere das Blutgefäß, eingestochen. Nach Herausziehen der inneren Nadel kann der Schlauch mit seinem Abgabeende voraus durch die Hülse in das Hohlorgan eingeschoben werden, bis das Abgabeende die gewünschte Stelle erreicht hat. In der Folge wird die Hülse entfernt. Die Hülse ist zu diesem Zweck entlang einer Aufreißlinie längs auftrennbar und somit vom Schlauch abnehmbar. In der Folge wird der Schlauch auf die gewünschte Länge abgelängt und auf einen Anschlussstutzen der Porteinheit aufgesteckt. Die Porteinheit wird in die vorbereitete Hauttasche eingesetzt und durch Festnähen an der Faszie fixiert.

Ein unvorteilhafter Schritt bei dieser Operationstechnik ist das Ablängen des Schlauches auf die korrekte Länge und das anschließende Zusammenstecken mit dem Anschlussstutzen der Porteinheit. Hierbei dürfen möglichst auch keine Luftblasen in den Schlauch gelangen. Auch das Abgabeende des Schlauches im Hohlorgan darf sich möglichst nicht verschieben. Zudem bedarf das Ablängen des Schlauches und das Zusammenstecken des abgelenkten Schlauches mit dem Anschlussstutzen eines intensiven Hantierens mit den Teilen des Portkatheters, wobei jede Berührung des Portkatheters durch den Operateur bzw. eine Berührung des Portkatheters mit der Haut des Patienten die Sterilität des Portkatheters mehr oder weniger beinträchtigen kann, was eine entsprechende Infektionsgefahr durch den implantierten Portkatheter mit sich bringt.

Aus der DE 8 437 873 U1 ist ein Portkatheter bekannt, bei dem die Länge des an die Porteinheit anschließenden Schlauches variierbar ausgebildet ist. Hierzu kann der Schlauch teilweise in der topfförmigen Porteinheit angeordnet und aus dem Anschlussstutzen der Porteinheit auf die gewünschte Länge ausziehbar sein. In einer anderen Ausführungsform ist der Schlauch um eine in einem Portgehäuse drehbar angeordnete Spule gewickelt und in der gewünschten Länge aus dem Portgehäuse ausziehbar. Durch diese Ausbildung kann zwar eine Vereinfachung der erforderlichen Manipulationen bei der Operation erreicht werden, wobei durch das erforderliche Hantieren am Portkatheter dennoch die Gefahr der Beeinträchtigung der Sterilität gegeben ist.

Portkatheter der eingangs genannten Art gehen aus der EP 1 736 195 A1 und EP 0 260 080 A2 hervor. Es ist jeweils ein Griffstück vorgesehen, welches mit der Porteinheit verbindbar und von dieser abnehmbar ist. Im verbundenen Zustand des Griffstücks mit der Porteinheit kann die Porteinheit mit Hilfe des Griffstücks manipuliert werden.

Aufgabe der Erfindung ist es einen vorteilhaften Portkatheter bereitzustellen, bei dem gegenüber Portkathetern, die kein abnehmbares Griffstück aufweisen, die Sterilität beim Implantieren verbessert ist. Erfindungsgemäß gelingt dies durch einen Portkatheter mit den Merkmalen des Anspruchs 1.

Bei der Implantation des Portkatheters kann somit die Porteinheit mittels des mit ihr verbundenen Griffstücks gehalten werden, sodass das Erfordernis die Porteinheit selbst zu berühren verringert oder vermieden ist. Nach dem Einsetzen der Porteinheit an die vorbereitete Stelle im Körper kann das Griffstück abgenommen werden. Das Griffstück ist somit zerstörungsfrei und vorzugsweise werkzeuglos von der Porteinheit trennbar, beispielsweise mittels eines beweglichen Betätigungselements des Griffstücks, über welches mindestens ein Verbindungselement des Griffstücks bewegbar ist, mit dem dieses Verbindungselement in ein Eingriffselement der Porteinheit eingreift. In einer möglichen Ausführungsform kann es sich um ein gegenüber einem Griffteil des Griffstücks verschiebbares Betätigungselement handeln, mittels dem das mindestens eine Verbindungselement z.B. aus dem zugeordneten Eingriffselement herausziehbar ist. Auch gegenüber einem Griffteil verschwenkbare oder verdrehbare Betätigungselemente können vorgesehen sein. Eine lösbare Kupplung des Griffstücks mit der Porteinheit kann in unterschiedlichen Formen ausgebildet sein.

Die von der Porteinheit abstehende Länge des Schlauches ist mittels dem mit der Porteinheit verbundenen Griffstück veränderbar, beispielsweise durch Drehung des Griffstücks oder eines Teils des Griffstücks um die Längsachse des Griffstücks.

In einer vorteilhaften Ausführungsform der Erfindung umfasst die Porteinheit einen Topf, der die innere Kammer zur Aufnahme der in das Hohlorgan des menschlichen oder tierischen Körpers einzubringenden Flüssigkeit aufweist, und ein Basisteil, gegenüber dem der Topf zur Veränderung der von der Porteinheit abstehenden Länge des Schlauches verdrehbar ist. Zur Veränderung der von der Porteinheit abstehenden Länge des Schlauches wird der Topf gegenüber dem Basisteil verdreht. Dadurch verläuft ein mehr oder weniger langer Abschnitt des Schlauches, der an das mit der Porteinheit verbundene Anschlussende des Schlauches anschließt, um einen Umfangsabschnitt des Topfes und die abstehende Länge des Schlauches verändert sich entsprechend.

Eine derartige Ausbildung des Portkatheters ermöglicht eine vorteilhafte Operationsmethode, bei der der bereits am Topf angebrachte Schlauch mit seinem Abgabeende voraus in das Hohlorgan eingeführt wird (z.B. entsprechend der Seldinger-Technik). Im Weiteren wird die am Griffstück gehaltene Porteinheit mittels des Griffstücks in die vorbereitete Stelle des menschlichen oder tierischen Körpers, insbesondere eine Hauttasche, eingesetzt, wobei vor oder nach oder sowohl vor als auch nach dem Einsetzen der Porteinheit die freie Länge des Schlauches durch Verdrehung des Topfes gegenüber dem Basisteil angepasst wird. Diese Anpassung kann hierbei insbesondere eine Verkürzung der freien Länge des Schlauchs umfassen. Vorteilhafterweise kann die Anpassung, insbesondere Verkürzung, der freien Länge des Schlauches mittels des Griffstücks erfolgen, insbesondere durch Drehung des Griffstücks oder eines Teils des Griffstücks um die Achse des Griffstücks.

Zur Verdrehung desTopfes gegenüber dem Basisteil weist das Griffstück vorteilhafterweise ein mit einem Getriebeglied des Topfes zusammenwirkendes Getriebeglied auf. Eine vorteilhafte Ausführungsform sieht hierbei zusammenwirkende Getriebeglieder in Form von Zahnrädern oder Ritzeln vor.

Ein bevorzugtes Verfahren zur Implantation des Portkatheters sieht vor, dass das Abgabeende des Schlauches zunächst etwas weiter in das Hohlorgan eingeführt wird, als es seiner vorgesehenen Endstellung entspricht. Nach dem Einsetzen der Porteinheit an die vorbereitete Stelle des menschlichen oder tierischen Körpers kann der Schlauch durch Verdrehen des Topfes gegenüber dem Basisteil unter Verkürzung der freien Länge des Schlauches wiederum etwas zurückgezogen werden, bis sich das Abgabeende an der vorgesehenen Stelle befindet. Die Verkürzung der freien Länge des Schlauches kann hierbei mittels des Griffstücks erfolgen, ebenso wie das Einsetzen der Porteinheit in die vorgesehene Stelle im Körper. Das Zurückziehen des Schlauches kann auch unter Röntgenkontrolle durchgeführt werden, sodass ein exaktes Platzieren des Abgabeendes erreicht wird. In der Folge wird das Griffstück von der Porteinheit abgenommen und die Öffnung im Körper, durch welche die Porteinheit eingeführt worden ist geschlossen. Die gesamte Implantation des Portkatheters kann auf diese Weise ohne direkte Berührung der Porteinheit durch den Operateur und vorzugsweise auch ohne direkte Berührung des Schlauches (der mit einem entsprechenden Instrument geführt werden kann) durch den Operateur durchgeführt werden.

Vorteilhafterweise kann der gesamte Portkatheter vor der Implantation mit Flüssigkeit, beispielsweise steriler Kochsalzlösung, bereits gefüllt werden. Ein Auftreten von Luftblasen kann dadurch effektiv vermieden werden.

Zur Befestigung der Porteinheit am Gewebe des menschlichen oder tierischen Körpers kann vorteilhafterweise das Basisteil mindestens ein in das Gewebe einstechbares Befestigungselement aufweisen. Diese Befestigung am Gewebe kann günstigerweise auch eine Gegenlagerung für die Drehung des Topfes gegenüber dem Basisteil mittels des Griffstücks bilden.

Bevorzugt ist es, wenn der Schlauch bereits werkseitig, also im Auslieferungszustand, mit dem Topf verbunden ist. Er kann hierbei günstigerweise seine größtmögliche freie Länge aufweisen und ausgehend von diesem Zustand mehr oder wenig weit in die Porteinheit zurückziehbar sein.

Weitere Vorteile und Einzelheiten der Erfindung werden im Folgenden anhand der beiliegenden Zeichnung erläutert. In dieser zeigen:
Fig. 1 eine Schrägsicht eines Portkatheters gemäß der Erfindung, wobei der Schlauch nur über einen Teil seiner Länge ausgezeichnet ist und ein Griffstück an der Porteinheit angebracht ist;
Fig. 2 eine Schrägsicht entsprechend Fig. 1 aus einer anderen Blickrichtung;
Fig. 3 und 4 Schrägsichten des Portkatheters aus verschiedenen Blickrichtungen, wobei Teile des Portkatheters nach Art einer Explosionsdarstellung auseinandergezogen dargestellt sind;
Fig. 5 einen Längsmittelschnitt durch den Portkatheter mit angebrachtem Griffstück;

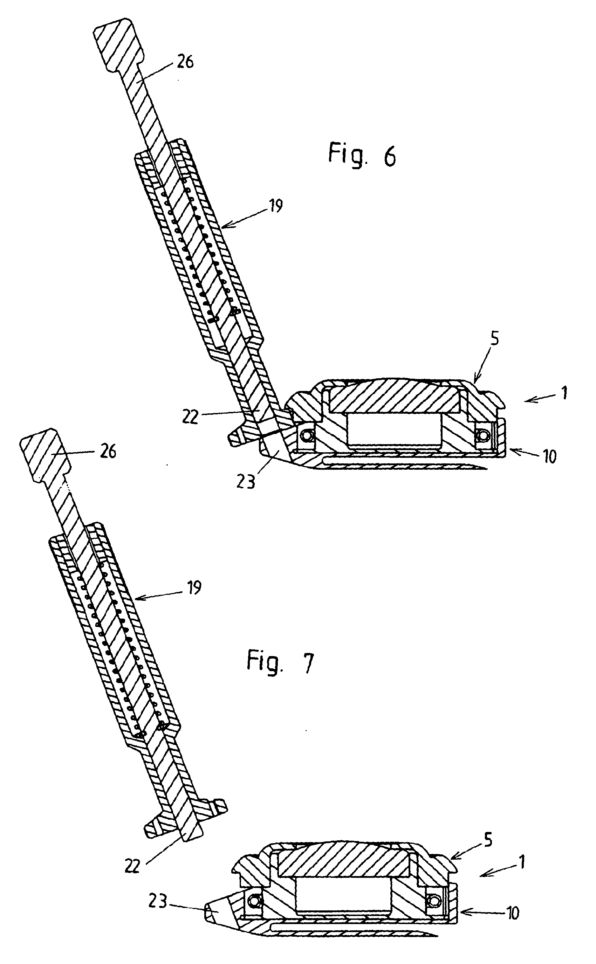

Fig. 6 einen Schnitt entsprechend Fig. 5, aber das Verbindungselement des Griffstücks zurückgezogen;
Fig. 7 einen Längsschnitt entsprechend Fig. 5, aber das Griffstück von der Porteinheit abgenommen;
Fig. 8 und 9 schematische Darstellungen zur Verdeutlichung der Implantation des Portkatheters.

Ein Ausführungsbeispiel eines Portkatheters gemäß der Erfindung, der auch als Infusionsport bezeichnet werden kann, ist in den Figuren dargestellt. Der Portkatheter umfasst eine Porteinheit 1 und einen an der Porteinheit 1 mit einem Anschlussende angeschlossenen Schlauch (=Katheterschlauch) 2. An seinem vom Anschlussende gegenüberliegenden Abgabeende 3 kann eine durch den inneren Kanal 4 des Schlauchs 2 fließende Flüssigkeit in ein Hohlorgan eines menschlichen oder tierischen Körpers eingebracht werden, wenn der Portkatheter in den menschlichen oder tierischen Körper implantiert ist.

Die Porteinheit 1 weist einen Topf 5 mit einer inneren Kammer 6 auf, in welche die in das Hohlorgan einzubringende Flüssigkeit einzuführen ist. Hierzu kann eine Membran 7, die einen Abschnitt der inneren Kammer 6 begrenzt, mit einer Injektionsnadel durchstochen werden, um die Flüssigkeit in die Kammer 6 einzuspritzen. Nach dem Herausziehen der Injektionsnadel schließt sich die Durchstichsöffnung der Membran 7 aufgrund der Elastizität des Materials der Membran 7, welches insbesondere aus Silikon bestehen kann.

Im gezeigten Ausführungsbeispiel ist die Membran 7 zwischen einem Topfunterteil 8 und einem Topfoberteil 9 eingesetzt, wobei das Topfoberteil 9 eine Öffnung aufweist, durch die hindurch die Membran 7 zugänglich ist.

Der innere Kanal 4 des Schlauchs 2 kommuniziert mit der Kammer 6 des Topfes 5. Beispielsweise ist der Schlauch 2 mit seinem Anschlussende auf einen Anschlussstutzen des Topfes 5 aufgesteckt, der eine in die Kammer 6 mündende Bohrung aufweist.

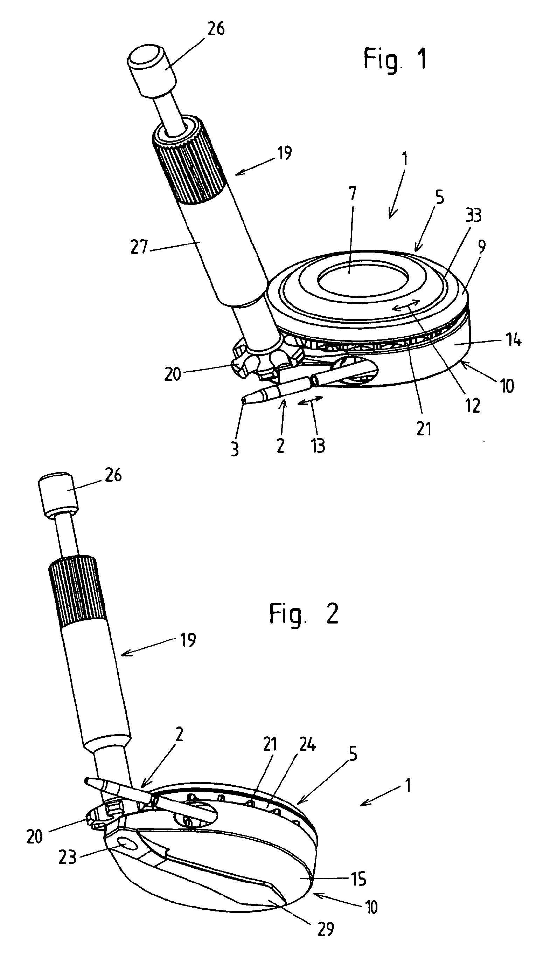

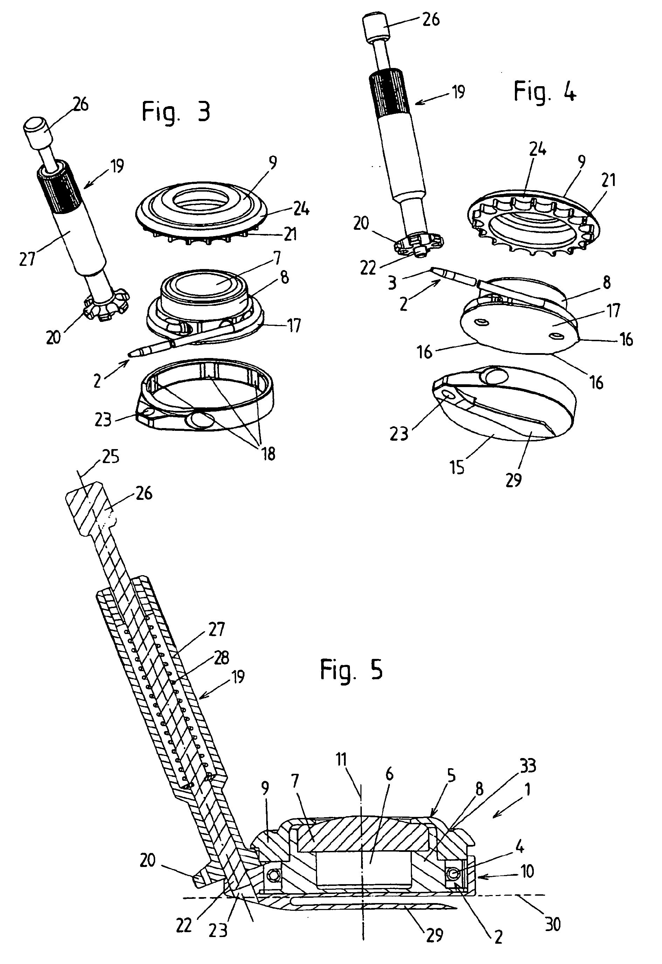

Die Porteinheit 1 weist weiters ein Basisteil 10 auf. Der Topf 5 ist gegenüber dem Basisteil 10 um eine Drehachse 11 (vgl. Fig. 5) verdrehbar. Es kann dadurch die vom Topf 5 bzw. von der Porteinheit 1 abstehende Länge des Schlauches 2 verändert werden. Hierzu ist ein an das Anschlussende anschließender Abschnitt des Schlauchs 2 um einen Umfangsabschnitt des Topfes 5 herumgeführt. Durch eine Verdrehung des Topfes 5 gegenüber dem Basisteil 10 kann dieser Umfangsabschnitt des Topfes 5, um den der Schlauch 2 um den Topf 5 verläuft und somit auch die freie Länge des Schlauches 2 verändert werden. Die möglichen Drehrichtungen des Topfes 5 gegenüber dem Basisteil 10 sind in Fig. 1 durch den Doppelpfeil 12 symbolisiert und die zugehörige Bewegung des Schlauches ist durch den Doppelpfeil 13 angedeutet.

Das Basisteil 10 weist einen kreisringförmigen Abschnitt 14 auf, mit dem es den äußeren ringförmigen Anlageabschnitt des Topfes 5 für den Schlauch 2 umgibt. Im kreisringförmigen Abschnitt 14 ist eine Durchtrittsöffnung für den Schlauch 2 ausgebildet, durch welche der Schlauch aus der Porteinheit 1 austritt.

Im gezeigten Ausführungsbeispiel weist das Basisteil 10 weiters einen Boden 15 auf, von dem der kreisringförmige Abschnitt 14 absteht. Ein solcher Boden könnte auch entfallen.

Das Basisteil 10 lagert den Topf 5 um die Drehachse 11 drehbar. Im gezeigten Ausführungsbeispiel können hierfür in der Drehstellung des Topfes 5, in welcher der Schlauch 2 am weitesten auf den Topf 5 aufgewickelt ist, Nasen 16 an einer Bodenplatte 17 des Topfunterteils 8 entlang von rillenförmigen Vertiefungen 18 des kreisringförmigen Abschnitts 14 bis zum Boden 15 des Basisteils 10 eingeschoben werden. Die Nasen 16 gelangen hierbei in eine Umfangsnut am unteren Ende des kreisringförmigen Abschnitts 14, welche die Verdrehung des Topfes 5 gegenüber dem Basisteil 10 unter Verlängerung der freien Länge des Schlauches ermöglicht. Nach der anfänglichen Verdrehung des Topfes 5 gegenüber dem kreisringförmigen Abschnitt 14 kann ein Rastteil überfahren werden, welches bei einem späteren Zurückdrehen des Topfes 5 als Anschlag wirkt, der bewirkt, dass der Topf 5 nicht mehr so weit zurückgedreht werden kann, dass die Nasen 16 in die zugeordneten Vertiefungen 18 gelangen. Ein Herausziehen des Topfes 5 aus dem Basisteil 10 wird somit verhindert.

Zum Manipulieren der Porteinheit 1 bei der Implantation des Portkatheters in den menschlichen oder tierischen Körper ist ein längliches Griffstück 19 vorhanden. Beim Implantieren des Portkatheters ist dieses an die Porteinheit 1 gekoppelt und steht von dieser ab, wie dies aus den Figuren 1, 2, 5, 8 und 9 ersichtlich ist.

Das Griffstück 19 weist ein Zahnrad 20 auf, welches mit einem am Topf 5 angeordneten Zahnrad 21 kämmt. Das Zahnrad 21 ist hierbei im gezeigten Ausführungsbeispiel einstückig mit dem Topfoberteil 9 ausgebildet. Andere Ausbildungen sind ebenfalls denkbar und möglich, beispielsweise eine Ausbildung am Topfunterteil 8 oder in Form eines separaten, am Topfoberteil 9 und/oder Topfunterteil 8 gehaltenen Teils.

Zur Verbindung des Griffstücks 19 mit der Porteinheit 1 dient weiters ein Verbindungselement 22 des Griffstücks 19, welches in ein Eingriffselement 23 des Basisteils 10 eingreift. Im gezeigten Ausführungsbeispiel wird das Verbindungselement von einem stiftförmigen Teil gebildet, welches in das von einem Loch gebildete Eingriffselement 23 eingreift. Das Verbindungselement 22 steht unterhalb des Zahnrads 20 über dieses vor und oberhalb des Zahnrads 21 ist ein Ringbund 24 ausgebildet (im gezeigten Ausführungsbeispiel am Topfoberteil), an dem sich das Zahnrad 20 abstützt.

In dieser Weise ist die Porteinheit 1 im mit dem Griffstück 19 gekoppelten Zustand an diesem gehalten.

Weiters ist in diesem gekoppelten Zustand durch Drehung des Griffstücks 19 um seine Längsachse 25 der Topf 5 gegenüber dem Basisteil 10 verdrehbar, wenn das Basisteil 10 gegenüber einer Drehung um die Längsachse 25 gehindert wird, insbesondere indem es am Gewebe des menschlichen oder tierischen Körpers festgelegt ist.

Zum Abnehmen des Griffstücks 19 von der Porteinheit 1 besitzt das Griffstück 19 ein Betätigungselement 26, mittels dem das Verbindungselement 22 zurückziehbar ist, um aus dem Eingriffselement 23 herausgezogen zu werden. Das Betätigungselement 26 wird im gezeigten Ausführungsbeispiel von einem hinteren Endabschnitt eines Bolzens gebildet, der ein Griffteil 27 des Griffstücks 19 durchsetzt und gegenüber diesem verschiebbar ist. Der vordere Endabschnitt dieses Bolzens bildet das Verbindungselement 22. Mittels einer Feder 28 ist der Bolzen in die Position vorgespannt, in der das Verbindungselement 22 über das Zahnrad 20 vorsteht.

Lösbare Kupplungen zur Verbindung des Griffstücks 19 mit der Porteinheit 1 sind in unterschiedlicher anderer Weise ausführbar.

Denkbar und möglich wäre es beispielsweise auch, dass nur ein Teil des Griffstücks zum Verdrehen des Topfes verdreht wird. Dieses Teil des Griffstücks wäre gegenüber dem Griffteil 27 drehbar gelagert (es könnte beispielsweise um den in den Fig. geriffelt dargestellten Abschnitt des Griffteils gebildet werden, der dann als separates drehbares Teil auszubilden wäre) und mit dem Zahnrad 20 oder einem anderen Getriebeglied zum Drehen des Topfes 5 drehfest verbunden. Das Griffteil 27 könnte in diesem Fall unverdrehbar gegenüber dem Basisteil 14 sein (beispielsweise durch eine mehreckige Ausbildung des Verbindungselements 22 und der das Eingriffselement 23 bildenden Öffnung und einer Verdrehsicherung des Verbindungselements 22 gegenüber dem Griffteil 27).

Anstelle von zusammenwirkenden Zahnrädern 20, 21 könnten auch andere Arten von zusammenwirkenden Getriebegliedern des Griffstücks 19 und des Topfes 5 zur Verdrehung des Topfes 5 gegenüber dem Basisteil 10 mittels des Griffstücks 19 vorgesehen sein, beispielsweise zusammenwirkende Reibräder.

Das Basisteil 10 weist zur Befestigung am Gewebe des menschlichen oder tierischen Körpers ein in das Gewebe einstechbares Befestigungselement 29 auf. Das Befestigungselement 29 ist hier als in das Gewebe geradlinig einstechbares schneidenförmiges Teil (Clip) ausgebildet. In anderer Form ausgebildete Befestigungselemente, beispielsweise hakenförmig ausgebildete Befestigungselemente sind denkbar und möglich. Es könnte auch mehr als ein solches Befestigungselement am Basisteil vorgesehen sein. Grundsätzlich denkbar und möglich wäre es auch, dass das Basisteil 10 Ösen zum Annähen des Basisteils am Gewebe aufweist.

Das Basisteil 10 ist somit in einer Anlageebene 30 am Gewebe befestigbar. Die Drehachse 11 des Topfes 5 gegenüber dem Basisteil 10 ist vorzugsweise rechtwinkelig zur Anlageebene 30 ausgerichtet.

Die Porteinheit 1 besitzt eine von der die Membran 7 freigebenden Öffnung beabstandete rinnenförmige Vertiefung 33, welche diese Öffnung umgibt. Es wird dadurch eine Abrutschsicherung für die Injektionsnadel ausgebildet, falls der behandelnde Arzt, der Flüssigkeit in die Porteinheit injizieren möchte, die die Membran 7 freigebende Öffnung verfehlen sollte. Diese Vertiefung 33 ist hier im Topfoberteil 9 angeordnet. Auch eine Anordnung im (entsprechend hochgezogenen) Topfunterteil 8 oder zwischen dem Topfoberteil 9 und dem Topfunterteil 8 ist denkbar und möglich. Anstelle einer einzelnen die Öffnung vollständig oder zumindest großteils umgebenden Vertiefung 33 könnten auch mehrere, die Öffnung insgesamt zumindest großteils umgebende Vertiefungen vorgesehen sein.

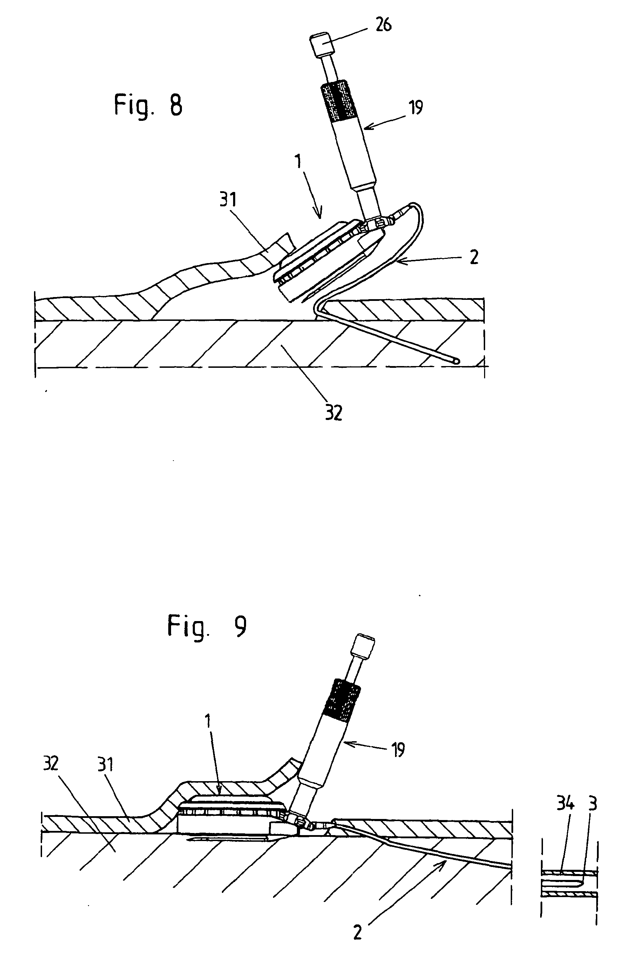

Üblicherweise wird die Porteinheit in eine ausgebildete Hauttasche implantiert. Diese Implantation ist in den Fig. 8 und 9 schematisch dargestellt. In Fig. 8 ist der Schlauch 2 bereits durch das Gewebe des menschlichen oder tierischen Körpers in das gewünschte Hohlorgan, beispielsweise eine Hohlvene eingebracht - beispielsweise nach Art der Seldinger-Technik - und eine Hautschicht (mit Unterhautfettgewebe) ist aufgeschnitten. Der Chirurg hebt die Hauttasche mit einer Pinzette auf und schiebt die Porteinheit 1 in diese ein, wobei das Befestigungselement 29 in die unter der Haut 31 liegende Faszie eingestochen wird. Diese Manipulationen der Porteinheit 1 können durchgeführt werden, indem der Chirurg das Griffstück 19 hält und mit diesem die am Griffstück 19 angebrachte Porteinheit 1 führt. In der Folge wird der Schlauch 2 durch Verdrehen des Topfes 5 mittels des Griffstücks 19 verkürzt, bis der in Fig. 9 dargestellte Zustand erreicht ist. Hierbei wird vorzugsweise auch der Schlauch 2 wieder ein Stück weit aus dem Gewebe herausgezogen, bis das Abgabeende 3 die vorgesehene Stelle im Hohlorgan erreicht hat. Dies geschieht unter Röntgenkontrolle.

Im Weiteren wird das Griffstück 19 von der Porteinheit durch Betätigung des Betätigungselements 26 abgenommen und die Hauttasche wird zugenäht. Die Implantation des Portkatheters ist auf diese Weise ohne Berührung der Porteinheit 1 durch den Handschuh des Chirurgen und vorzugsweise auch ohne Berührung des Schlauches 2 durch den Handschuh des Chirurgen erfolgt.

### Legende

### zu den Hinweisziffern:

- 1: Porteinheit
- 2: Schlauch
- 3: Abgabeende
- 4: innerer Kanal
- 5: Topf
- 6: innere Kammer
- 7: Membran
- 8: Topfunterteil
- 9: Topfoberteil
- 10: Basisteil
- 11: Drehachse
- 12: Doppelpfeil
- 13: Doppelpfeil
- 14: kreisringförmiger Abschnitt
- 15: Boden
- 16: Nase
- 17: Bodenplatte
- 18: Vertiefung
- 19: Griffstück
- 20: Zahnrad
- 21: Zahnrad
- 22: Verbindungselement
- 23: Eingriffselement
- 24: Ringbund
- 25: Längsachse
- 26: Betätigungselement
- 27: Griffteil
- 28: Feder
- 29: Befestigungselement
- 30: Anlageebene
- 31: Haut
- 32: Faszie
- 33: Vertiefung

## Patentansprüche

1. Portkatheter zum Einbringen einer Flüssigkeit in ein Hohlorgan eines menschlichen oder tierischen Körpers, mit einer in den menschlichen oder tierischen Körper implantierbaren Porteinheit (1), die eine innere Kammer (6) aufweist, welcher die in das Hohlorgan des menschlichen oder tierischen Körpers einzubringende Flüssigkeit zuführbar ist, und einem an die Porteinheit (1) angeschlossenen Schlauch (2), der einen inneren Kanal (4) aufweist, durch den hindurch die Flüssigkeit in das Hohlorgan leitbar ist, wobei zum Manipulieren der Porteinheit (1) bei der Implantation des Portkatheters in den menschlichen oder tierischen Körper ein mit der Porteinheit (1) verbundenes und von dieser abnehmbares Griffstück (19) vorgesehen ist, **dadurch gekennzeichnet, dass** die von der Porteinheit (1) abstehende Länge des Schlauches (2) mittels des mit der Porteinheit (1) verbundenen Griffstücks (19) veränderbar ist.

2. Portkatheter nach Anspruch 1, **dadurch gekennzeichnet, dass** die von der Porteinheit (1) abstehende Länge des Schlauchs (2) durch Drehung des Griffstücks (19) oder eines Teils des Griffstücks (19) um eine Längsachse (25) des Griffstücks (19) veränderbar ist.

3. Portkatheter nach Anspruch 1 oder 2, **dadurch gekennzeichnet, dass** die Porteinheit (1) einen Topf (5), der die innere Kammer (4) aufweist, und ein Basisteil (10) umfasst, gegenüber dem der Topf (5) zur Veränderung der von der Porteinheit (1) abstehenden Länge des Schlauchs (2) verdrehbar ist.

4. Portkatheter nach Anspruch 3, **dadurch gekennzeichnet, dass** Griffstück (19) im mit der Porteinheit (1) verbundenen Zustand mindestens ein in ein Eingriffselement (23) des Basisteils (10) eingreifendes Verbindungselement (22) aufweist.

5. Portkatheter nach Anspruch 4, **dadurch gekennzeichnet, dass** zum Abnehmen des Griffstücks (19) von der Porteinheit (1) das Griffstück (19) ein Betätigungselement (26) aufweist, mittels dem das oder mindestens eines der Verbindungselemente (22) des Griffstücks (19) bewegbar ist.

6. Portkatheter nach einem der Ansprüche 3 bis 5, **dadurch gekennzeichnet, dass** das Griffstück (19) ein mit einem Getriebeglied des Topfs (5) zusammenwirkendes Getriebeglied aufweist.

7. Portkatheter nach Anspruch 6, **dadurch gekennzeichnet, dass** die zusammenwirkenden Getriebeglieder Zahnräder (20, 21) sind.

8. Portkatheter nach einem der Ansprüche 3 bis 7, **dadurch gekennzeichnet, dass** das Basisteil (10) zur Befestigung am Gewebe des menschlichen oder tierischen Körpers mindestens ein in das Gewebe einstechbares Befestigungselement (29) aufweist.

9. Portkatheter nach einem der Ansprüche 1 bis 8, **dadurch gekennzeichnet, dass** die Porteinheit (1) eine Membran (7) aufweist, die zum Zuführen der Flüssigkeit in die Kammer (6) von einer Injektionsnadel durchstechbar ist.

10. Portkatheter nach einem der Ansprüche 1 bis 9, **dadurch gekennzeichnet, dass** der Schlauch (2) bereits werkseitig mit der Porteinheit (1) verbunden ist.

11. Portkatheter nach einem der Ansprüche 3 bis 10, **dadurch gekennzeichnet, dass** das Basisteil (10) einen kreisringförmigen Abschnitt (14) aufweist, mit dem es den Topf (5) umgibt.

## Claims

1. A port catheter for the introduction of a fluid into a hollow organ of a human body or animal body, having a port unit (1), implantable into the human or animal body, which has an inner chamber (6) to which there can be fed the fluid to be introduced into the hollow organ of the human or animal body, and having a hose (2), attached to the port unit (1), which has an inner channel (4) through which the fluid can be conducted into the hollow organ, wherein for manipulation of the port unit (1) upon implantation of the port catheter into the human or animal body there is provided a handle piece (19) which is connected to the port unit (1) and is detachable therefrom, **characterised in that** the length of hose (2) projecting from the port unit (1) can be altered by means of the handle piece (19) connected to the port unit (1).

2. A port catheter according to claim 1, **characterised in that** the length of hose (2) projecting from the port unit (1) can be altered by rotation of the handle piece (19) or of part of the handle piece (19) about a longitudinal axis (25) of the handle piece (19).

3. A port catheter according to claim 1 or 2, **characterised in that** the port unit (1) comprises a pot (5), having the inner chamber (4), and a base part (10) with respect to which the pot (5) can be rotated in order to alter the length of hose (2) projecting from the port unit (1).

4. A port catheter according to claim 3, **characterised in that** in its state in which it is connected to the port unit (1), handle piece (19) has at least one connecting element (22) engaging an engagement element (23) of the base part (10).

5. A port catheter according to claim 4, **characterised in that** the handle piece (19) has an operating element (26) for detachment of the handle piece (19) from the port unit (1), by means of which operating element (26) there can be moved the connecting element (22), or at least one connecting element (22), of the handle piece (19).

6. A port catheter according to any one of claims 3 to 5, **characterised in that** the handle piece (19) has a gear member cooperating with a gear member of the pot (5).

7. A port catheter according to claim 6, **characterised in that** the cooperating gear members are toothed wheels (20, 21).

8. A port catheter according to any one of claims 3 to 7, **characterised in that** for attachment to the tissue of the human or animal body, the base part (10) has at least one securing element (29) insertable into the tissue.

9. A port catheter according to any one of claims 1 to 8, **characterised in that** the port unit (1) has a membrane (7) through which a hypodermic needle can be pierced in order to feed the fluid into the chamber (6).

10. A port catheter according to any one of claims 1 to 9, **characterised in that** connection of the hose (2) to the port unit (1) takes place at the factory.

11. A port catheter according to any one of claims 3 to 10, **characterised in that** the base part (10) has a circular portion (14) by means of which it surrounds the pot (5).

## Revendications

1. Cathéter à chambre implantable pour le transport d'un liquide vers un organe creux d'un organisme humain ou animal, avec une unité de chambre (1) implantable dans l'organisme humain ou animal, dotée d'une chambre interne (6) où peut être injecté le liquide à transporter vers l'organe creux de l'organisme humain ou animal, et avec une tubulure (2) reliée à l'unité de chambre (1), laquelle est dotée d'un canal interne (4) par lequel le liquide peut être acheminé dans l'organe creux, une pièce de préhension (19) raccordée à l'unité de chambre (1) et amovible de celle-ci étant prévue pour la manipulation de l'unité de chambre (1) lors de l'implantation du cathéter à chambre implantable dans l'organisme humain ou animal, **caractérisé en ce que** la longueur de tubulure (2) dépassant de l'unité de chambre (1) est modifiable au moyen de la pièce de préhension (19) raccordée à l'unité de chambre (1).

2. Cathéter à chambre implantable selon la revendication 1, **caractérisé en ce que** la longueur de tubulure (2) dépassant de l'unité de chambre (1) est modifiable par rotation de la pièce de préhension (19) ou d'une partie de la pièce de préhension (19) autour d'un axe longitudinal (25) de la pièce de préhension (19).

3. Cathéter à chambre implantable selon la revendication 1 ou la revendication 2, **caractérisé en ce que** l'unité de chambre (1) comprend un boîtier (5) doté de la chambre intérieure (4), et une pièce de base (10) rotative par rapport au boîtier (5) afin de modifier la longueur de tubulure (2) dépassant de l'unité de chambre (1).

4. Cathéter à chambre implantable selon la revendication 3, **caractérisé en ce qu'**en état de raccordement à l'unité de chambre (1), la pièce de préhension (19) comporte au moins un élément de raccordement (22) s'engageant dans un élément de logement (23) de la pièce de base (10).

5. Cathéter à chambre implantable selon la revendication 4, **caractérisé en ce que**, pour le retrait de la pièce de préhension (19) de l'unité de chambre (1), la pièce de préhension (19) comporte un élément de manipulation (26) au moyen duquel la pièce de préhension (19), ou au moins un des éléments de raccordement (22) de la pièce de préhension (19) sont déplaçables.

6. Cathéter à chambre implantable selon l'une des revendications 3 à 5, **caractérisé en ce que** la pièce de préhension (19) comporte un élément d'engrenage coopérant avec un élément d'engrenage du boîtier (5).

7. Cathéter à chambre implantable selon la revendication 6, **caractérisé en ce que** les éléments d'engrenage coopérant entre eux sont des roues dentées (20, 21).

8. Cathéter à chambre implantable selon l'une des revendications 3 à 7, **caractérisé en ce que**, pour la fixation sur un tissu de l'organisme humain ou animal, la pièce de base (10) comprend au moins un élément de fixation (29) pouvant percer le tissu.

9. Cathéter à chambre implantable selon l'une des revendications 1 à 8, **caractérisé en ce que** l'unité de chambre (1) comprend une membrane (7) perforable par une aiguille pour injection pour la conduction du liquide dans la chambre (6).

10. Cathéter à chambre implantable selon l'une des revendications 1 à 9, **caractérisé en ce que** la tubulure (2) est raccordée à l'unité de chambre (1) départ usine.

11. Cathéter à chambre implantable selon l'une des revendications 3 à 10, **caractérisé en ce que** la pièce de base (10) comporte une partie en forme de couronne (14) par laquelle elle entoure le boîtier (5).
